# EUROPEAN PATENT SPECIFICATION

(11) **EP 2 881 056 B1**
(45) Date of publication and mention of the grant of the patent: **30.05.2018**
(21) Application number: 14196976.6
(22) Date of filing: 09.12.2014
(51) Int. Cl.: A61B 17/70

(54) **Extension device for a bone anchor, in particular for minimally invasive surgery**
Verlängerungsvorrichtung eines Knochenankers, insbesondere für die minimalinvasive Chirurgie
Dispositif d'extension pour ancrage osseux, en particulier pour la chirurgie effractive minimale

(30) Priority: 09.12.2013 US 201361913777 P; 09.12.2013 EP 13196326; 17.06.2014 US 201462013415 P
(43) Date of publication of application: 10.06.2015
(73) Proprietor: Biedermann Technologies GmbH & Co. KG, 78166 Donaueschingen (DE)
(72) Inventor: Biedermann, Timo, 78647 Trossingen (DE); Dannecker, Berthold, 78112 St. Georgen (DE)
(74) Representative: Prüfer & Partner mbB Patentanwälte · Rechtsanwälte

(56) References cited:
- WO-A2-2013/112689
- US-A1- 2012 022 594
- US-B1- 8 211 110
- US-B1- 8 439 922

## Description

The invention relates to an extension device for a bone anchor, in particular for use in minimally invasive surgery (MIS). The invention also relates to a system including such an extension device and a bone anchor, wherein the bone anchor comprises an anchoring section and a receiving part for receiving a rod to couple the rod to the anchoring section. The extension device includes a first sleeve and a second sleeve positioned within the first sleeve and a coupling structure that allows to couple the extension device to the receiving part of the bone anchor and that inhibits a translational and rotational movement of the extension device relative to the receiving part.

Extension devices, also called head extenders, for pedicle screws for use in minimally invasive surgery are known in the art. For example, US 7,563,264 B2 describes a spinal stabilization system for a minimally invasive procedure wherein detachable sleeves may be coupled to a collar of a bone anchor to allow for formation of the spinal stabilization system through a small skin incision. The detachable sleeve members may allow for alignment of the collars to facilitate insertion of an elongated member in the collars. A coupling system is provided between the sleeve and the collar that inhibits translational movement of the sleeve relative to the collar. In one embodiment, the sleeve may be coupled to a collar of a bone fastener assembly with movable members that may be threaded into threaded openings in the collar.

WO 2013/112689 A2 describes a minimally invasive tower access device comprising an elongated outer sleeve that slidably receives an elongated inner sleeve. A lock nut is used to secure the inner sleeve and outer sleeve in a locked mode.

US 8,439,922 B1 discloses systems and methods for a guide assembly for introducinh a bone anchor to an operative target site. The guide assembly comprises an outer sleeve, an inner sleeve and a sleeve extension coupled to a cap. The cap allows rotating the sleeve extension to advance the inner sleeve distally. The sleeve extension interacts with the outer sleeve via threaded regions. The outer sleeve has lateral projection tabs which engage a recess provided at the housing.

US 8,211,110 B1 discloses a minimally invasive tool to facilitate implanting a pedicle screw and a corresponding housing. The tool has an outer sleeve, an inner sleeve and a connector that pushes inner sleeve and interacts with the outer sleeve via threads. The outer sleeve has tabs which include mating surfaces which engage corresponding surfaces on the housing. The inner sleeve has alignment portions which mate with corresponding alignment detents at the housing.

It is the object of the invention to provide an improved extension device for a bone anchor, in particular for use in minimally invasive surgery, and a system of such an extension device and a bone anchor that facilitates the surgical steps and improves the safety of the surgical measures such as compression and distraction.

The object is solved by a system according to claim 1. Further developments are given in the dependent claims.

The extension device is configured to be coupled to the receiving part such that it is locked against translational and rotational movement of the extension device relative to the receiving part. Because translational and rotational movements of the extension device and the receiving part are inhibited, the connection between the extension device and the receiving part is robust. This permits a safe placement of the rod and a set screw for fixing the rod as well as the following surgical steps of adjustment of the spinal stabilization system, such as compression or distraction. These steps can be performed using the extension device attached to the receiving part.

The extension device comprises a first sleeve and a second sleeve positioned within the first sleeve and a coupling member that connects the second sleeve to the first sleeve and allows a controlled axial movement of the second sleeve relative to the first sleeve. In some embodiments, after the extension device has been attached to the receiving part, the first sleeve and the second sleeve can be interlocked with each other and with the receiving part by operating the coupling member which is an interlocking bushing in one direction. The interlocking connection between the first sleeve and the second sleeve can be released by operating the interlocking bushing in the opposite direction.

A translational movement between the extension device and the receiving part can be inhibited by a form-fit engagement of a circumferential rib that extends at least partially around the longitudinal axis of the device and engages a corresponding circumferential groove. The rib may be provided at the first sleeve and the groove at the receiving part.

A rotational movement between the extension device and the receiving part may be inhibited by the engagement of a longitudinal rib that engages a longitudinal groove. The rib may be provided at the first sleeve and the groove at the receiving part. The form-fit connection of the longitudinal ribs and grooves allows to apply a high torque.

The extension device comprises only few parts which facilitates the assembly and operation of the device.

Further features and advantages of the invention will become apparent from the description of embodiments using the accompanying drawings.

In the drawings:
- Fig. 1: shows a perspective exploded view of the extension device according to a first comparative example.
- Fig. 2: shows a perspective view of the extension device of Fig. 1 in an assembled state.
- Fig. 3: shows a perspective view of a first comparative example of a receiving part of a bone anchor that may form together with the extension device of Figs. 1 and 2 a first comparative example of a system of an extension device and a bone anchor.
- Fig. 4: shows a top view of the receiving part of Fig. 3.
- Fig. 5a: shows a cross-sectional view of the receiving part of Fig. 3 along line A-A in Fig. 4.
- Fig. 5b: shows an enlarged view of a detail of Fig. 5a.
- Fig. 6: shows a perspective view of a first sleeve of the extension device of Figs. 1 and 2.
- Fig. 7: shows a perspective view from the bottom of a front end portion of the first sleeve of Fig. 6.
- Fig. 8: shows a top view of the first sleeve of Fig. 6.
- Fig. 9: shows a cross-sectional view of the first sleeve of Fig. 6 along line B-B in Fig. 8.
- Fig. 10: shows a cross-sectional view of the first sleeve of Fig. 6 along line D-D in Fig. 9.
- Fig. 11a: shows a perspective view of a second sleeve of the extension device of Figs. 1 and 2.
- Fig. 11b: shows an enlarged perspective view of a detail of Fig. 11a.
- Fig. 12: shows a cross-sectional view of the second sleeve according to Fig. 11a, the cross section taken in a plane containing a longitudinal sleeve axis and extending through the center of the legs of the sleeve.
- Fig. 13: shows a perspective view of an interlocking bushing of the extension device of Figs. 1 and 2.
- Fig. 14: shows a cross-sectional view of the interlocking bushing of Fig. 13, the section taken in a plane containing a cylinder axis of the bushing.
- Fig. 15: shows a perspective view of a first step of assembling the extension device of Figs. 1 and 2, wherein the interlocking bushing of Fig. 13 is to be mounted to the rear end section of the second sleeve of Fig. 11a.
- Fig. 16: shows a perspective view of the interlocking bushing and the first sleeve of Fig. 15 mounted together.
- Figs. 17 to 19a: show cross-sectional views of steps of attaching the extension device of the first comparative example to a polyaxial bone anchor.
- Fig. 19b: shows an enlarged view of a detail of Fig. 19a.
- Fig. 20: shows a cross-sectional view of the bone anchor with attached extension device according to the first comparative example in a state before the extension device is rotationally locked with respect to the bone anchor.
- Fig. 21: shows a cross-sectional view of an upper portion of the extension device in the state shown in Fig. 20.
- Fig. 22: shows an enlarged cross-sectional view of the bone anchor extension device in a state in that the extension device is rotationally locked with respect to the bone anchor.
- Fig. 23: shows a cross-sectional view of the upper portion of the extension device in the state shown in Fig. 22.
- Fig. 24: shows a perspective view of a receiving part according to a first embodiment that forms together with an extension device according to first embodiment first embodiment of a system of an extension device and a bone anchor.
- Fig. 25: shows an enlarged perspective view of a front end portion of first sleeve of the extension device according to the first embodiment.
- Fig. 26: shows a perspective view of the front end portion of the extension device according to the first embodiment with a bone anchor having a receiving part according to the first embodiment shown in Fig. 24.
- Fig. 27: shows an enlarged perspective view of a front end portion of a second sleeve of the extension device according to the first embodiment.
- Figs. 28 to 30a: show cross-sectional views of steps of attaching the extension device according to Figs. 25 to 27 to a polyaxial bone anchor.
- Fig. 30b: shows an enlarged view of a detail of Fig. 30a.
- Fig. 31: shows an enlarged perspective view of a modified embodiment of a second sleeve of an extension device.
- Fig. 32: shows a perspective view of a front end portion of a still further modified embodiment of the second sleeve.
- Fig. 33: shows a perspective view of a section of the spinal column with inserted pedicle screws and attached extension devices according to the invention.
- Fig. 34: shows a perspective view of a receiving part according to a second embodiment that forms together with an extension device according to a second embodiment a second embodiment of a system of an extension device and a bone anchor.
- Fig. 35: shows an enlarged perspective view of a front end portion of the first sleeve of the extension device according to the second embodiment.
- Fig. 36: shows a perspective view of the front end portion of the extension device according to the second embodiment with a bone anchor having a receiving part according to the second embodiment shown in Fig. 34.
- Fig. 37: shows a top view of the extension device according to the second embodiment.
- Fig. 38: shows an enlarged cross-sectional view of the front end portion of the extension device according to the second embodiment with a bone anchor having a receiving part according to the second embodiment along lines D-D' in Fig. 37.

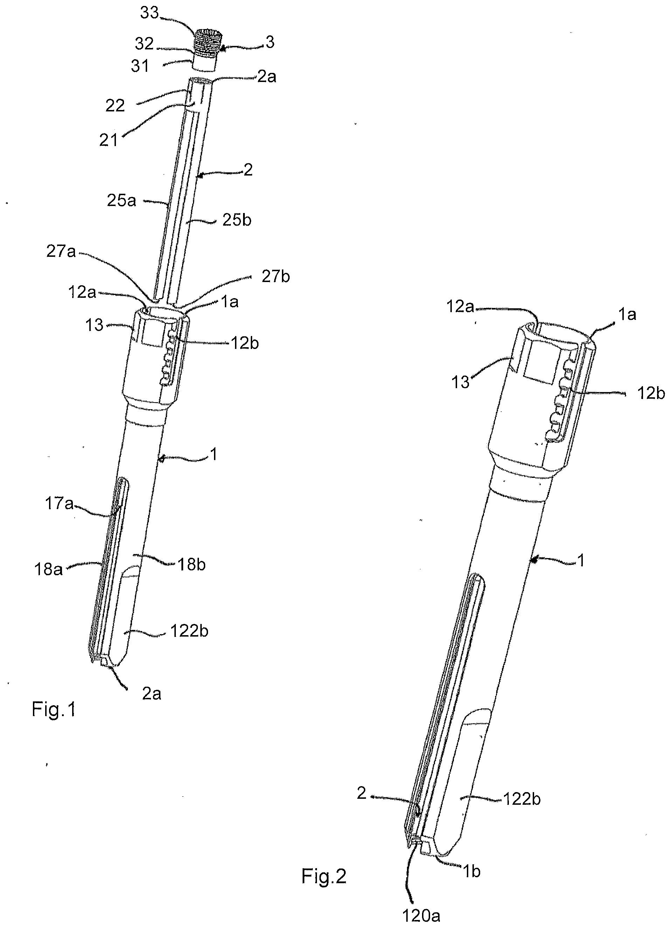

Referring to Figs. 1 and 2, an extension device according to a first comparative example comprises a first sleeve 1 that forms an outer sleeve, a second sleeve 2 that forms an inner sleeve and that is positionable within the first sleeve 1 and an interlocking bushing 3. The interlocking bushing 3 is configured to be connected to the second sleeve 2 and is configured to couple the second sleeve 2 to the first sleeve 1 and to permit a controlled motion of the second sleeve 2 relative to the first sleeve 1.

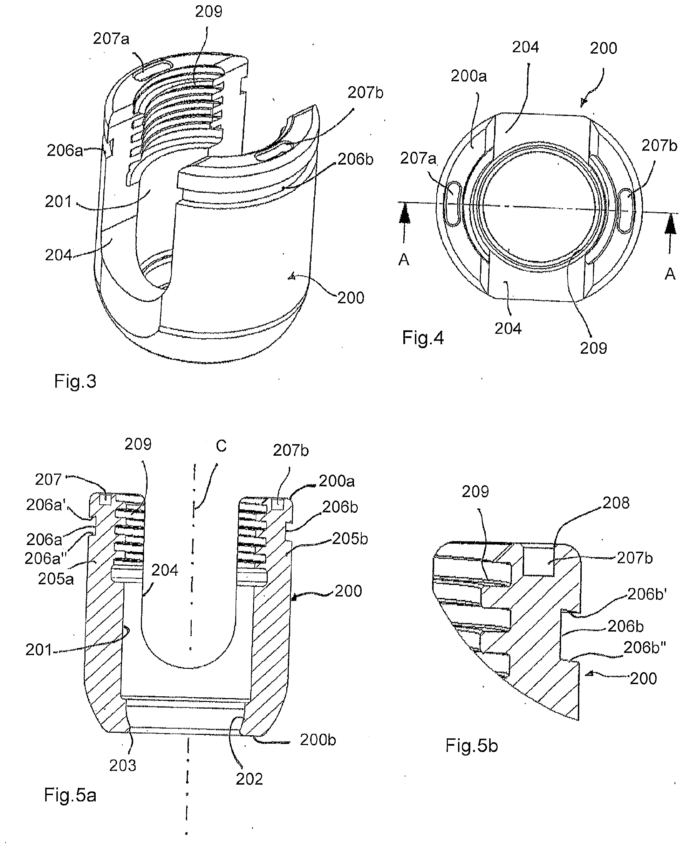

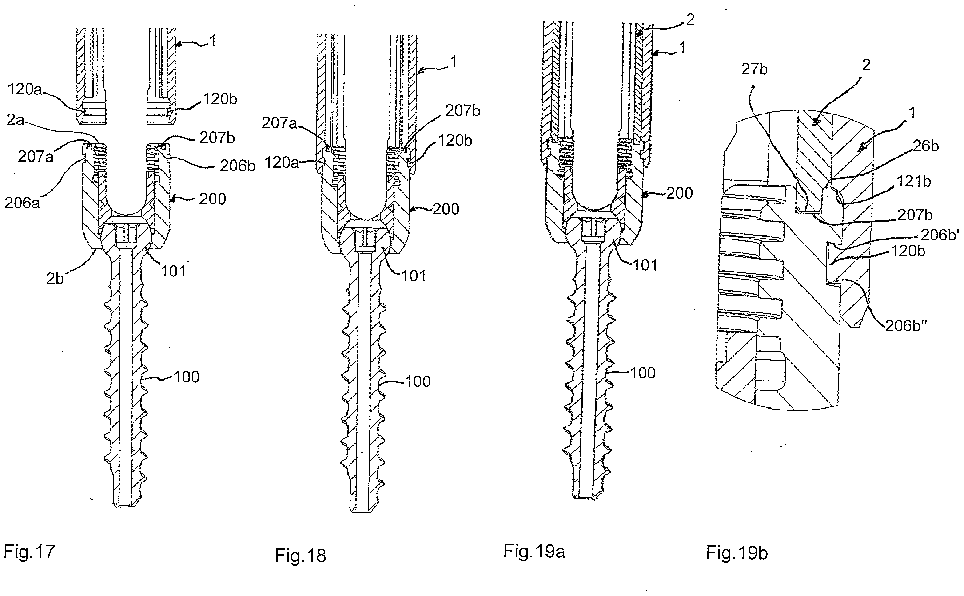

The extension device shown in Figs. 1 and 2 is configured to be used with a bone anchor that shall be explained first. An example of a such a bone anchor comprises an anchoring element with threaded shank 100 and a spherical segment shaped head 101, as shown in Figs. 17 to 19a wherein the anchoring element is pivotably coupled to a receiving part 200. The receiving part 200 is shown more in detail in Figs. 3 to 5b. The receiving part 200 typically is a substantially cylindrical part with a first end or top end 200a and a second or bottom end 200b, a central axis C, a coaxial bore 201 extending from the top end 200a to a distance from the bottom end 200b, a seat 202 for the head 101 of the anchoring element and a lower opening 203 at the bottom end through which the shank of the bone anchoring element can pass through. A substantially U-shaped recess 204 extends from the top end 200a in the direction of the bottom end 200b. The recess 204 serves for receiving a rod (not shown). By means of the recess 204 two free legs 205a, 205b are formed. In the outer surface of the legs 205a, 205b at a distance from the top end 200a, circumferentially extending grooves 206a, 206b are formed that extend from one end of the channel formed by the U-shaped recess 204 to the other end and that are open towards the U-shaped recess 204. An upper side wall 206a', 206b' and a lower side wall 206a", 206b" of the groove 206a, 206b may be inclined towards the bottom end 200b.

The receiving part 200 further comprises at the top end 200a in a free end surface of each of the legs 205a, 205b a recess 207a, 207b that extends into the legs in a direction coaxial to the central axis (C). The recess 207a, 207b is in a top view elongated and closed in a circumferential direction at both end. As can be seen in particular in Fig. 5b, a cross-section of the recesses 207a, 207b is substantially square or may be rectangular. The recesses 207a, 207b serve for engagement with a portion of the extension device as described below. Towards the top end 200a there may be a chamfered section 208 provided at each recess 207a, 207b that facilitates engagement of the recess with the corresponding protrusion at the extension device.

In an upper portion of the legs, an internal thread 209 is provided for cooperation with a locking screw (not shown) for fixing the rod.

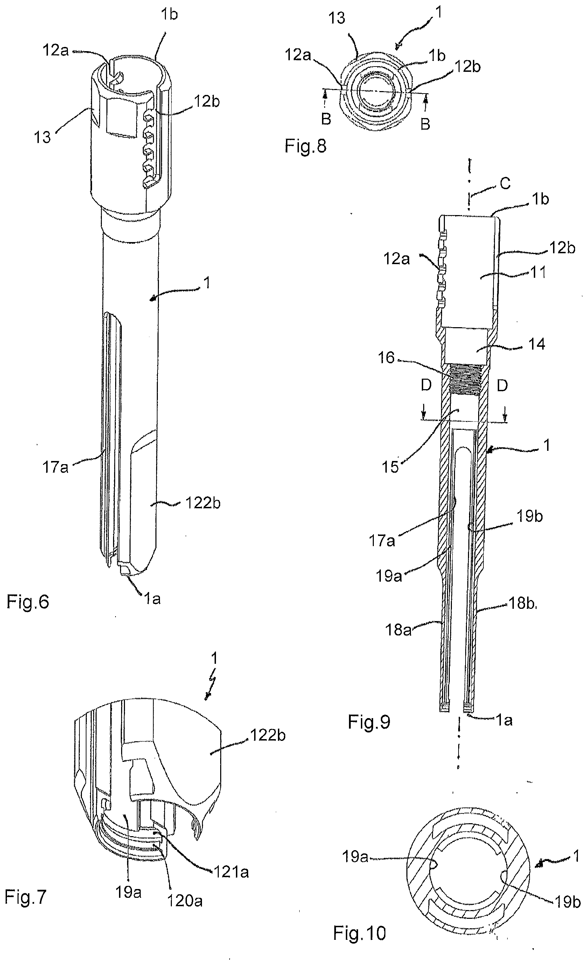

Referring to Figs. 6 to 10, the first sleeve 1 of the extension device comprises a longitudinal axis c that is coaxial with the central axis C of the receiving part 200 when the extension device is coupled to the receiving part. The first sleeve 1 further has a front end 1a and a rear end 1b.

The first sleeve 1 comprises adjacent to the rear end 1b a first section 11 with a largest inner diameter compared to other sections of the first sleeve 1. Two coaxial slits 12a, 12b extend from the second end 1b along at least a portion of the first section 11 in a longitudinal direction. One of the sidewalls of each longitudinal slit 12a, 12b comprises a wavy structure as can be seen in particular in Fig. 6 for latching with a reduction sleeve (not shown) used for further steps in the surgical procedure, for example for pressing down the rod and inserting a locking screw to fix the rod. Furthermore, an engagement structure 13, for example, a plurality of flat engagement portions, is provided at an outer surface of the first section 11 that form an engagement portion 13 for engagement with a tool. Following the first section 11, the first sleeve 1 comprises a second section 14 with slightly smaller inner diameter than the first section 11 and adjacent thereto a third section 15 with a smaller inner diameter compared to the second section 14 and first section 13. In the third section, an internal thread 16 is provided that is configured to cooperate with the interlocking bushing 3 as described below.

Two elongate substantially U-shaped slits 17a, 17b which have a reverse U-shape compared to the slit 204 in the receiving part and that are offset from each other by 180° extend from the front end 1a toward the rear end 1b up to a distance from the second section 14 of the first sleeve. The longitudinal slits 17a, 17b have a width in circumferential direction that is greater than a diameter of the rod and have the function of permitting the rod to be inserted therethrough. The width of the slits 17a, 17b may be substantially the same as the width of the U-shaped recess 204 in the receiving part 200. By means of the slits 17a, 17b, the first sleeve 1 comprises two free legs 18a, 18b that are intended to cooperate with the free legs 205a, 205b of the receiving part 200. The inner surface of the third section 15 of the first sleeve 1 comprises at each of the legs 18a, 18b a longitudinally extending substantially cylinder segment-shaped guiding recess 19a, 19b into which a portion of the second sleeve can extend to be guided therein. The guiding recesses 19a, 19b extend in a longitudinal direction beyond the longitudinal recesses 17a, 17b toward the rear end 1b, as can be seen in Fig. 9.

Each of the legs 18a, 18b comprises at a distance from the front end 1a a projection 120a, 120b that extends in a circumferential direction around the longitudinal axis c from one slit 17a to the opposite slit 17b. The shape of the circumferential projection 120a, 120b is substantially complementary to the shape of the circumferential grooves 206a, 206b of the receiving part, as can be seen in particular in Fig. 19b. An upper surface of the projection 120a, 120b that faces toward the rear end 1b may be inclined toward the rear end to match the complementary inclined upper wall 206a', 206b' of the groove 206a, 206b. A lower surface of the circumferential projection 120a, 120b may be perpendicular to the central axis or also inclined toward the rear end 1b. Between the projection 120a, 120b in the end of the recesses 19a, 19b an inner surface portion 121a, 121b substantially matches an outer surface portion of the receiving part 200 between the grooves 206a, 206b and the top end 200a.

By means of the legs 18a, 18b, the first sleeve is slightly flexible in a radial direction so that the first sleeve 1 can be snapped onto the legs 205a, 205b of the receiving part 200.

An outer diameter of the first sleeve 1 may vary between the rear end 1b and the front end 1a. The first sleeve 1 may have a greatest outer diameter in the first section 11 followed by one or more successively decreasing outer diameters towards the front end 1a as shown in Fig. 9. In addition, flattened outer surface portions 122a, 122b may be provided at an outer surface of the legs 18a, 18b that extend from the front end 1a toward the rear end 1b for cooperation with a tool (not shown).

The total length of the first sleeve is such that when the bone anchor is inserted into the bone and the first sleeve is attached to the receiving part 200, the rear end and first section 11 are sufficiently above the operation site when the extension device is used.

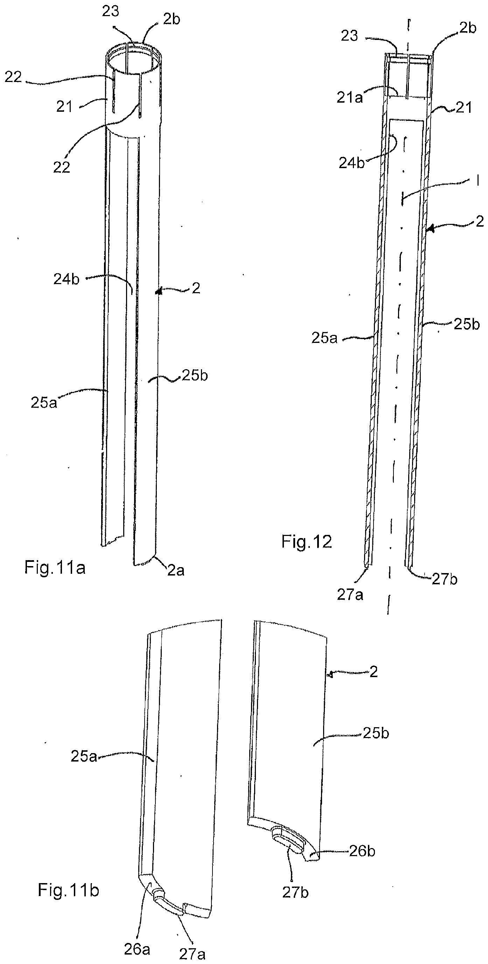

Referring now to Figs. 11a to 12, the second sleeve 2 comprises a front end 2a and an opposite rear end 2b. The second sleeve 2 has a substantially constant outer diameter. Adjacent to the rear end 2b, the second sleeve 2 comprises a first portion 21 with a circumferentially closed cylinder surface, wherein the first portion 21 is configured to engage the interlocking bushing 3. A plurality of coaxially extending slits 22 that are open end to the rear end 2b and that extend to a distance from the rear end 2b are provided to render the first portion 21 flexible in such a way that the first portion 21 can elastically snap onto a portion of the interlocking bushing 3 and is held there by friction. At a first distance from the rear end 2b a circumferentially extending annular projection 23 projecting inward is provided that cooperates with a corresponding depression or recess at the interlocking bushing 3 to inhibit an inadvertent removal of the interlocking bushing 3 from the second sleeve 2. At a second distance from the rear end 2b a stop 21a, for example in the form of an annular shoulder is provided that limits the insertion of the interlocking bushing 3 and provides an abutment for the interlocking bushing 3 when the interlocking bushing is screwed into the first sleeve 1. An outer diameter of the first section 21 of the second sleeve 2 is such that is fits into third section 15 of the first sleeve 1.

Two recesses 24a, 24b with substantially rectangular cross section extend from the front end 2a through the second sleeve 2 up to the first portion 21. The recesses have such a size that two opposite legs 25a, 25b are formed that fit into the guiding recesses 19a, 19b of the first sleeve 1. The legs 25a, 25b have such a length that they extend beyond the upper closed end of the recesses 17a, 17b of the first sleeve 1 in a direction toward the rear end 1b of the first sleeve 1.

The front end 2a of the second sleeve 2 is comprised of a substantially flat surface portion 26a, 26b on each of the legs 25a, 25b that is configured to cooperate with a substantially flat surface portion on the top end 200a of the receiving part, and a projection 27a, 27b on each leg, that is configured to cooperate with the recess 207a, 207b at the free end of the legs of the receiving part 200. Hence, the projection 27a, 27b has a complementary shape to the shape of the recess 207a, 207b of the receiving part. As can be seen in Fig. 11b, a side surface of the projection 27a that faces toward the longitudinal axis of the second sleeve 2 is flush with the inner surface of the legs 25a, 25b. An outer surface of the projections 27a, 27b is slightly recessed with respect to an outer surface of the legs 25a, 25b. The shape of the projections 27a, 27b is substantially arc shaped with rounded edges like the corresponding recess 207a, 207b in the receiving part. In a circumferential direction, the projection 27a, 27b are arranged substantially in the middle of the legs 25a, 25b.

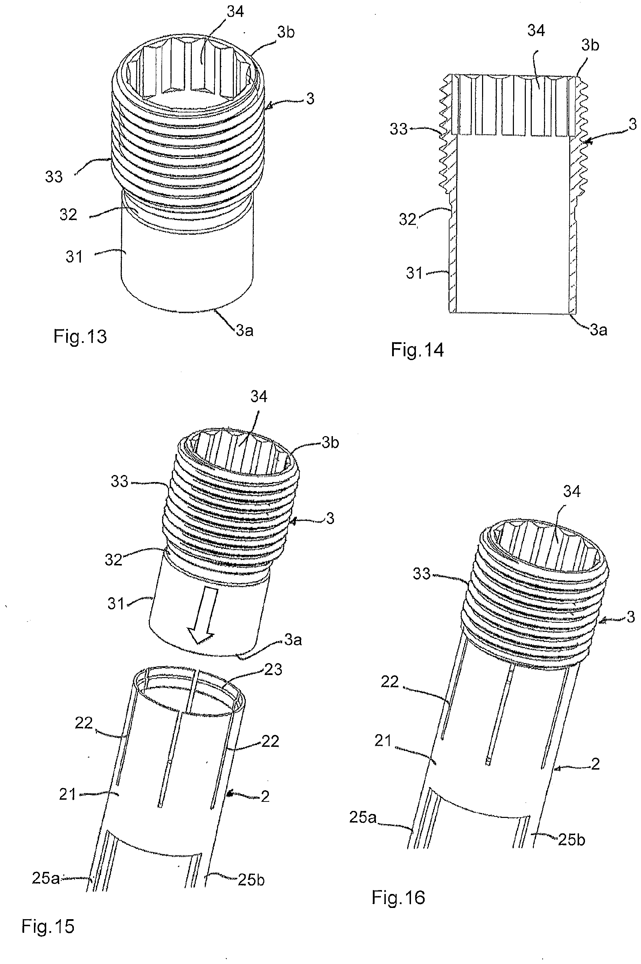

Turning now to Figs. 13 and 14, the interlocking bushing 3 comprises a front end 3a and a rear end 3b and a cylindrical section 31 that is adjacent to the front end 3a and that has an outer diameter that is substantially the same as an inner diameter of the first section 21 of the second sleeve 2. The size of the inner portion of the first section 21 of the second sleeve 2 and the cylindrical section 31 of the bushing 3 is such that the bushing 3 is held in the second sleeve 2 by friction. Adjacent to the cylindrical section 31 with a smooth outer surface, there is a groove 32 for engaging with the annular projection 23 of the second sleeve 2. Adjacent to the rear end 3b, a threaded portion 33 with an external thread is provided that is configured to cooperate with the threaded section 16 of the first sleeve 1. An inner diameter of the bushing 3 may be substantially constant and sized such that a locking member for fixing the rod in the receiving part can be inserted therethrough. Adjacent to the rear end 3b, the inner surface of the bushing 3 comprises an engagement portion 34, for example, a plurality of longitudinally extending grooves and projections for engaging with a tool.

The assembly of the second sleeve 2 and the interlocking bushing 3 is shown in Fig. 15 and 16. The interlocking bushing is pushed with its cylindrical portion 31 into the first section 21 of the second sleeve 2 until the inwardly projecting annular projection 23 snaps into the groove 32 of the interlocking bushing 3. By means of this, the interlocking bushing 3 is coupled to the second sleeve 2 in such a manner, that a rotational motion of the interlocking bushing 3 relative to the second sleeve 2 is possible. An axial movement of the interlocking bushing 3 relative to the second sleeve 2 is inhibited when the interlocking bushing abuts against the stop 21a provided in the second sleeve 2. The connection between the second sleeve 2 and the first sleeve 1 via the interlocking bushing is a rigid connection.

The assembly consisting of the second sleeve 2 and the interlocking bushing 2 as shown in Fig. 16 is then inserted into the first sleeve 1 from the rear end 1b until the threaded portion 33 of the interlocking bushing engages the threaded section 16 of the first sleeve 1. The legs 25a, 25b are guided in the guiding recesses 19a, 19b in the first sleeve 1. By means of this, the extension device is assembled.

Referring now to Figs. 17 to 23, the attachment of the extension device to a bone anchor will be described. As shown in Fig. 17, the extension device is moved with the front end 1a of the first sleeve 1 toward the receiving part 200. Then, as depicted in Fig. 17, the legs 18a, 18b of the first sleeve are spread to a certain extent when they touch the top end 200a of the receiving part 200 and by further downward movement of the extension device, the circumferential projections 120a, 120b of the legs 18a, 18b of the first sleeve 1 snap into the circumferential grooves 206a, 206b that are provided at the receiving part 200. As soon as the projections have engaged the recesses, an axial movement of the first sleeve 1 in a direction away from the receiving part 200 is inhibited.

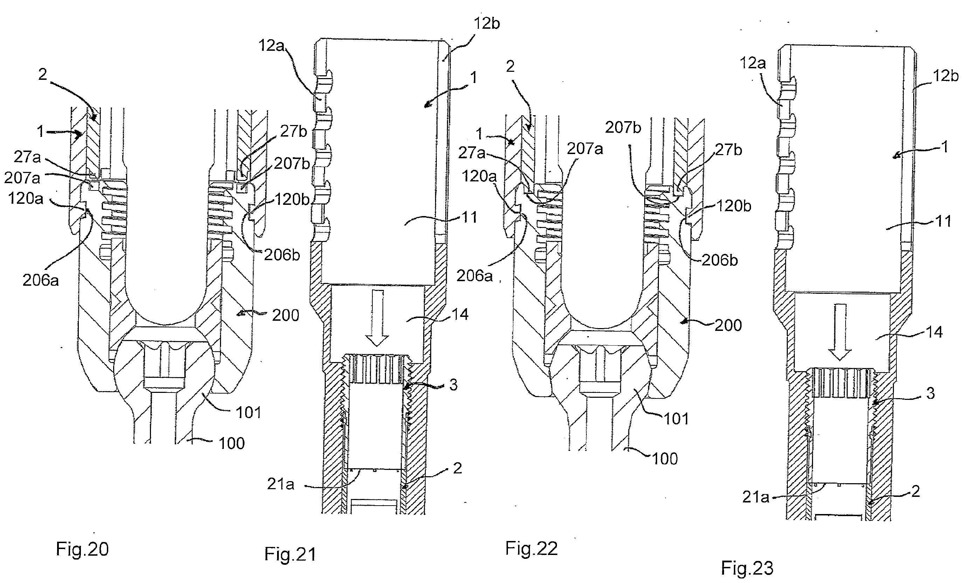

Then, as shown in Figs. 19a and 19b, the second sleeve 2 is moved relative to the first sleeve 1 toward the receiving part 200 by screwing the interlocking bushing 3 further towards the front end 1a of the first sleeve 1. When the projections 27a, 27b of the second sleeve 2 enter the corresponding recesses 207a, 207b at the top end 200a of the receiving part 200 a rotational movement of the second sleeve 2 relative to the first sleeve 1 and therefore relative to the receiving part 200 is inhibited. Referring to Figs. 20 and 21, a step of moving the second sleeve 2 relative to the first sleeve 1 is shown. In Fig. 20, the position of the second sleeve 2 relative to the first sleeve 1 which is already attached to the receiving part 200 is such that the projections 27a, 27b are above the recesses 207a, 207b of the receiving part. The interlocking bushing extends slightly into the second section 14 of the first sleeve 1. In Fig. 22 the projections 27a, 27b have entered the recesses 207a, 207b fully. This is achieved, as shown in Fig. 23, by rotating the interlocking bushing 3 so that it advances in the threaded section 16 of the first sleeve 1. Because the interlocking bushing 3 abuts against the stop 21a in the second sleeve 2, the interlocking bushing 3 pushes the second sleeve 2 forward when it moves. The second sleeve 2 can rotate relative to the bushing 3 so that the alignment between the legs 18a, 18b of the first sleeve 1 and the legs 25a, 25b of the second sleeve 2 is maintained. A further rotation of the interlocking bushing 3 presses the flat surface portion 26a, 26b of the front end 2a of the second sleeve 2 onto the free flat end surfaces of the receiving part 200. Through this and the engagement of the projections 120a, 120b with the grooves 206a, 206b the first sleeve 1 is interlocked with the receiving part 200 and with the second sleeve 2 to provide a safe and strong connection between the extension device and the receiving part. In such a configuration, the insertion of the rod and locking screw can take place as well as surgical steps thereafter such as compression and distraction steps thereby using the extension device.

Turning the interlocking bushing 3 in the opposite direction releases the interlocking connection and permits to retract the projection 27a, 27b of the second sleeve 2 out of the recesses 207a, 207b. The extension device may be detached from the receiving part 200 by first removing the second sleeve 2 together with the interlocking bushing 3 and then using a tool for detaching the first sleeve such that the circumferential projection 120a, 120b disengage from the grooves 206a, 206b of the receiving part 200.

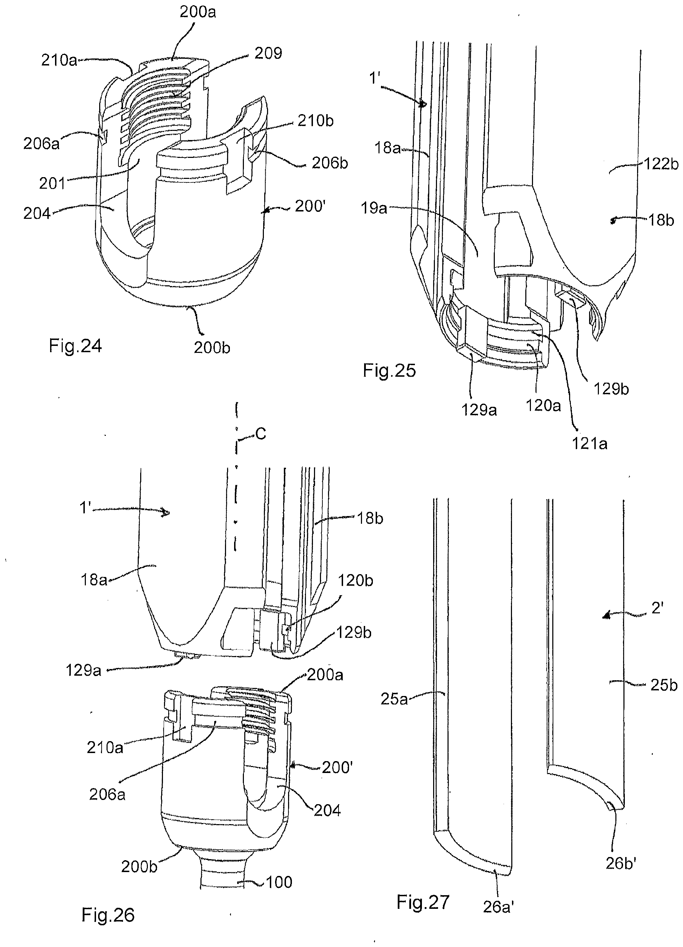

A first embodiment of the extension device is shown in Fig. 24 to 27. All parts and portions of the first embodiment that are identical or similar to that of the first comparative example are marked with the identical reference numerals and the description thereof is not repeated. The first embodiment of the extension device differs from the first comparative example by the design of the front portion of the first sleeve and the second sleeve. Also the receiving part differs from the receiving part of the first comparative example.

The extension device according to the first embodiment comprises a first sleeve 1', a second sleeve 2' and the interlocking bushing 3 that is the same as in the first comparative example. As can be seen in Fig. 24, the receiving part 200' comprises in addition to the circumferential grooves 206a, 206b on each leg 205a, 205b a coaxially extending recess 210a, 210b that is open towards the top end 200a. The recesses 210a, 210b are provided in the outer surface of the legs 205a, 205b and do not completely extend through the wall of the legs 205a, 205b. The position of the recesses 210a, 210b is substantially in the center of each leg 205a, 205b. Furthermore, the recesses 210a, 210b extend through and beneath the circumferential grooves 206a, 206b. The contour of the recesses 210a, 210b is substantially rectangular, but any other shape that achieves the result of providing an abutment against a rotational motion of the first sleeve 1' may be contemplated.

Referring to Figs. 25 and 26, the first sleeve 1' comprises two projections 129a, 129b that are extending from the front end 1a toward the longitudinal axis c and that are positioned, sized and shaped complementary to the recesses 210a, 210b of the receiving part 200'. As depicted in Fig. 26, the projections 129a, 129b project slightly over the arc shaped end surface of each leg 18a, 18 b of the first sleeve 1'. The engagement of the projections 129a, 129b with the corresponding coaxial recesses 210a, 210b of the receiving part 200' generates a form fit connection that is configured to inhibit a rotational movement of the first sleeve 1' relative to the receiving part 200'.

The second sleeve 2' differs from the second sleeve 2 of the first comparative example in that the projections 27a, 27b are omitted. Hence, the front end 2a of the second sleeve 2' comprises only two arc shaped flat surfaces 26a', 26b' corresponding to each leg 25a, 25b as shown in Fig. 27.

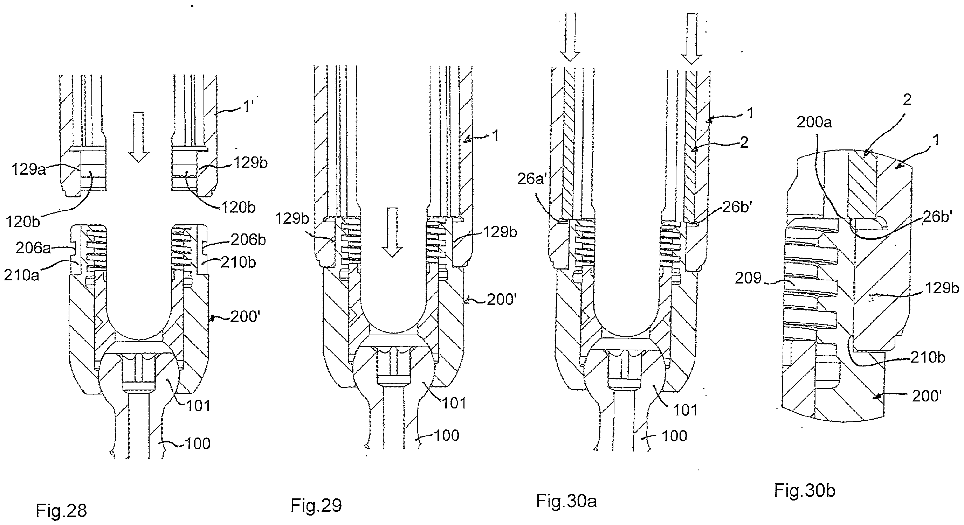

The attachment of the extension device to the receiving part 200' will be explained with reference to Figures 28 to 30b. First, as shown in Fig. 28, the extension device according to the first embodiment is moved with its front end 1a of the first sleeve 1' toward the receiving part 200' in such a manner that the legs 18a, 18b of the first sleeve 1' are aligned with the legs 205a, 205b of the receiving part 200'. A further downward movement of the first sleeve 1' causes an engagement of the coaxial projections 129a, 129b of the first sleeve 1' with the coaxial recesses 210a, 210b of the receiving part 200' as well as a snap in of the circumferential projections 120a, 120b into the circumferential grooves 206a, 206b of the receiving part 200'. Once the grooves and the recesses are in full engagement, an axial and a rotational movement of the first sleeve 1' relative to the receiving part 200' is inhibited. Thereafter, the second sleeve 2' is advanced toward the receiving part 200' by rotating the interlocking bushing 3 until the free end surfaces 26a', 26b' abut against the free end surface 200a of the first end of the receiving part 200' as shown in detail in Fig. 30b. Final tightening of the interlocking bushing 3 generates a force-fit connection between the abutting surfaces of the second sleeve 2' and the receiving part 200' and an interlocking of the extension device relative to the receiving part. Through rotating the interlocking bushing 3 in the opposite direction the connection is released.

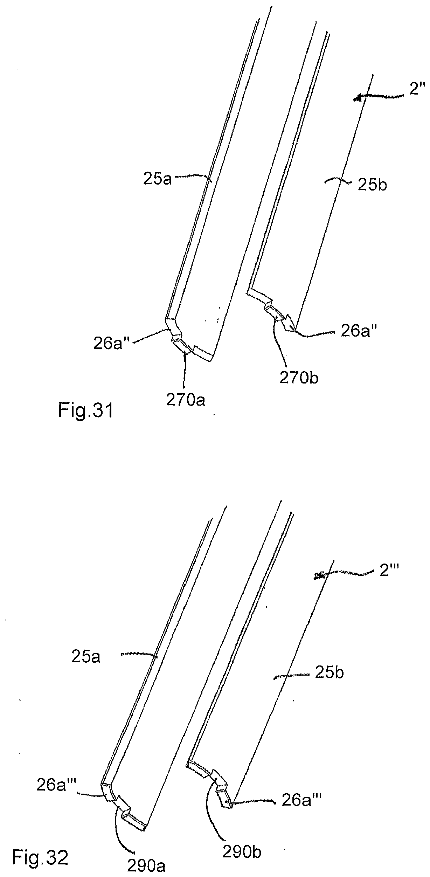

A modified embodiment of the design of the second sleeve is shown in Fig. 31. The second sleeve 2" may have at its front end 2a instead of the projection 27a, 27b according to the first comparative example projections 270a, 270b that are flush with the inner and outer surface of the legs 25a, 25b and have an arc-shape with a flat front surface and perpendicular side walls or inclined side walls. The projections 270a, 270b may be easy to manufacture in that just a portion of the leg 25a, 25b is cut away. A corresponding receiving part has complementary shaped recesses for engagement with the projections 270a, 270b.

A still further modified embodiment of the second sleeve is shown in Fig. 32. The second sleeve 2'" comprises at its front end 2a two recesses 290a, 290b on the legs 25a, 25b. A corresponding receiving part (not shown) comprises a complementary shape to achieve a form-fit connection.

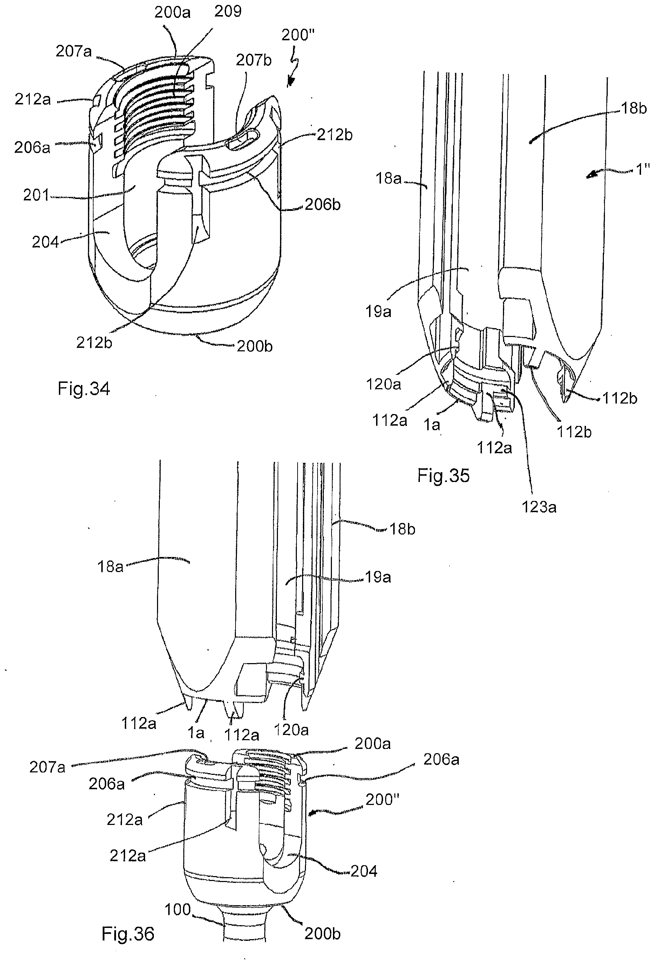

A second embodiment of the extension device and of a system comprising the extension device and a receiving part of a polyaxial bone anchor is shown in Figs. 34 to 38. A receiving part 200" that is adapted to cooperate with the extension device is similar to the receiving part 200 of the first comparative example. Parts that are identical or highly similar to the corresponding parts of the first comparative example are indicated with the same reference numerals and the description thereof is not repeated. The receiving part 200" comprises a pair of longitudinal grooves 212a at one side of the U-shaped recess 204 and a pair of longitudinal grooves 212b at the opposite side. The grooves of each pair of grooves 212a, 212b are spaced apart from each other in the circumferential direction. Furthermore, the grooves extend from the top end 200a in the outer wall of the receiving part on either side of the recesses 207a, 207b. The grooves 212a, 212b are open towards the top end 200a and have a decreasing depth towards their closed end that is located at a distance from the top end 200a. The cross-section of the longitudinal grooves 212a, 212b is substantially rectangular. As can be seen in particular in Figs. 34 and 36, the longitudinal grooves 212a, 212b intersect the circumferentially extending grooves 206a, 206b. The purpose of the longitudinal grooves 212a, 212b is to cooperate with corresponding longitudinally extending pairs of ribs of the first sleeve in order to provide a form-fit connection.

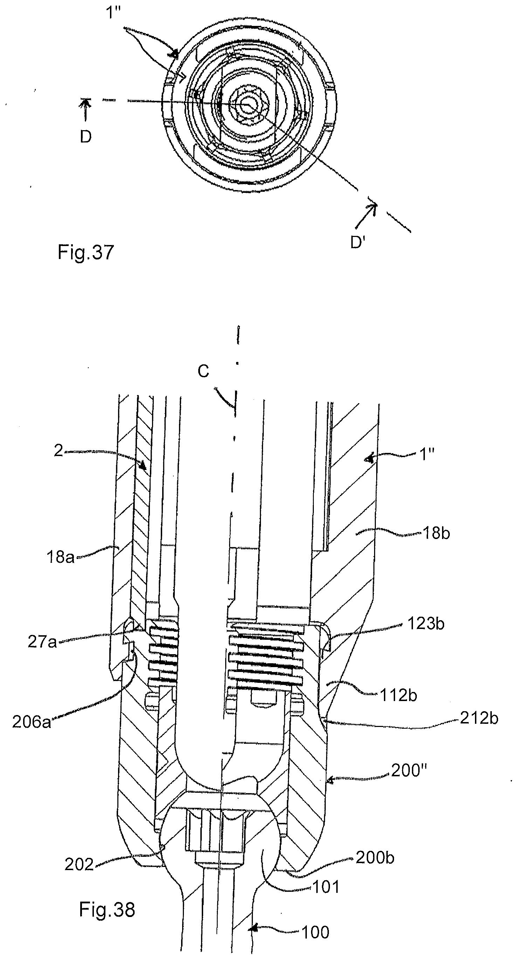

The extension device according to the second embodiment comprises a first sleeve 1" that is similar to the first sleeve 1 of the first comparative example. In the following, only the differences will be explained. The first sleeve 1" comprises at positions, that correspond in a circumferential direction to the positions of the longitudinal grooves 212a, 212b, a pair of ribs 112a and a pair of ribs 112b on the legs 18a, 18b. The ribs 112a, 112b protrude inwards from the legs 18a, 18b and downwards from the front end 1a and have a shape that substantially matches the shape of the grooves 212a, 212b. As can be seen in particular in Fig. 38, an outer surface of the ribs 112a, 112b tapers towards the free end, such that, when seen in a circumferential direction, the cross-section of the ribs is substantially triangular.

From each of the longitudinal ribs 112a, 112b a transverse thickened rib portion 123a, 123b extends outward in a circumferential direction. This transverse rib portion 123a, 123b is located at a distance from the front end 1a that corresponds to the distance of the circumferentially extending groove 206a, 206b from the top end 200a. Hence, the transverse rib portion 123a, 123b is at the same level as the circumferential projection 120a, 120b.

The second sleeve may be identical to the second sleeve 2 of the first comparative example that has the projections 27a, 27b for engaging the recesses 207a, 207b on the top end 200a of the receiving part 200".

By means of the longitudinal ribs 112a, 112b that engage the longitudinal grooves 212a, 212b, respectively, the strength of the connection between the head extension device and the receiving part is enhanced. The longitudinal ribs and grooves allow to apply a high torque with the extension device to the receiving part.

In use, the first sleeve 1" is snapped over the receiving part 200" until the form-fit connection between the circumferential projection 120a, 120b with the transverse portion 123a, 123b and the circumferential groove 206a, 206b is established. The longitudinal ribs 112a, 112b engage the longitudinal grooves 212a, 212b. Thereby, rotation between the head extension device and the receiving part is prevented.

It shall be noted that for the second sleeve 2 also the second sleeve 2', 2", 2"' of the second embodiment may be used. The number of cooperating longitudinal ribs and grooves may vary. It should be at least one rib and one groove on each leg but can be also more than two.

The parts of the extension device are all made of a body-compatible material, such as titanium or stainless steel, a body-compatible metal alloy, for example Ti-Ni alloy, such as Nitinol, or a body-compatible plastic material, such as PEEK.

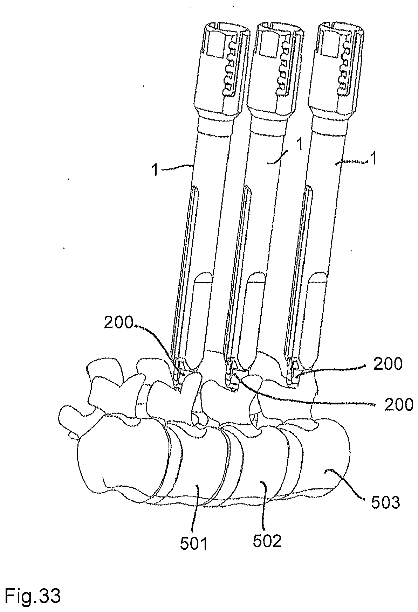

In the clinical use, as shown in Fig. 33, an extension device according to each of the embodiments described above is attached to a receiving part 200 of a bone anchor that has been inserted into a pedicle of a vertebra 501, 502, 503. The legs of the sleeves of the extension device are aligned with the legs of the receiving part such that by rotating the extension devices the channels of the receiving parts of the bone anchors can be aligned to permit the insertion of a rod. Because the connection between the receiving part and the extension device is robust and safe, an easy alignment using the extension devices is possible. Thereafter, the rod is inserted (not shown) and fixed with a locking screw that is guided through the first and second sleeve of the extension device until it can be screwed between the legs 205a, 205b of the receiving part. By applying an instrument to the extension device, a compression or distraction procedure can be performed using a minimally invasive technique.

Modifications of the above described embodiments may be contemplated. It shall be noted, that the shape of the engaging complementary structures of the first sleeve and the receiving part as well as the second sleeve and the receiving part can be modified and are not limited to the exact shape shown in the embodiments.

The extension device can be used with any bone anchor that comprises a receiving part, such as polyaxial bone anchor, a monoaxial bone anchor and to different shapes of receiving parts. Anchors with inner compression members or outer rings may be used. The only necessity is the engagement structure at an upper end of the receiving part that cooperates with a corresponding structure of the extension device.

## Claims

1. A system of an extension device and a bone anchor,
wherein the bone anchor comprises:
an anchoring section (100) for anchoring in a bone and
a receiving part (200', 200") connected to the anchoring section, the receiving part comprising a central axis (C) and a channel (204) for receiving a rod, wherein sidewalls of the channel form two free legs (205a, 205b), and
wherein the extension device comprises:
a first sleeve (1', 1 ") with a first sleeve axis (c) that is coaxial with the central axis (C), wherein the first sleeve (1', 1") is configured to contact and be detachably coupled to the receiving part (200', 200"), and
a second sleeve (2', 2", 2"') with a second sleeve axis, wherein the second sleeve is positioned within the first sleeve (1', 1 ") and is configured to be coupled to the receiving part so as to hinder or inhibit a rotational movement of the second sleeve (2', 2", 2"') relative to the receiving part (200', 200"), wherein the second sleeve (2', 2", 2"') is connected to the first sleeve (1, 1') through a coupling member (3) that is configured to advance in the first sleeve (1', 1") together with the second sleeve (2', 2", 2"') in an axial direction;
wherein the first sleeve (1', 1 ") comprises a circumferentially extending protrusion (120a, 120b) at an inner surface thereof that is configured to engage a complementary structure of a circumferentially extending groove (206a, 206b) at an outer surface of the receiving part (200', 200") such as to inhibit a translational movement of the first sleeve (1', 1") relative to the receiving part along the central axis,
wherein the first sleeve (1', 1") further comprises at least one longitudinally extending rib (112a, 112b) or protrusion (129a, 129b) that engages a corresponding longitudinally extending groove (212a, 212b, 210a, 210b) formed at one of the legs of the receiving part (200', 200") so as to also inhibit a rotational movement of the receiving part relative to the first sleeve (1', 1") **characterised in that** the longitudinal grooves (212a, 212b, 210a, 210b) intersect the circumferentially extending grooves (206a, 206b) to provide a form-fit connection between the first sleeve (1', 1") and the receiving part (200', 200").

2. The system of claim 1, wherein the the coupling member (3) is coupled to the first sleeve (1', 1 ") through an advancement structure (33, 16) that permits to advance the coupling member with the second sleeve (2', 2", 2"') to a given position.

3. The system of claim 1 or 2, wherein the coupling member (3) is a bushing and the advancement structure comprises threads (33, 16).

4. The system of one of claims 1 to 3, wherein the coupling member (3) is coupled to the second sleeve (2', 2", 2"') via an abutment (21a) of the second sleeve (2', 2", 2"') against which the coupling member (3) abuts, wherein rotation of the coupling member (3) with respect to the second sleeve (2', 2", 2"') is allowed.

5. The system of one of claims 1 to 4, wherein the second sleeve (2", 2"') comprises a protrusion (27a, 27b; 270a, 270b) and/or a recess (290a, 290b) at a surface thereof that is configured to engage a complementary structure at a surface of the receiving part to provide a form-fit connection between the second sleeve and the receiving part.

6. The system of one of claims 1 to 5, wherein the second sleeve (2') comprises a substantially flat surface portion (26a', 26b') that is configured to engage a substantially flat surface portion (200a) at the receiving part (200') such that a friction between the surface portions is generated when the first sleeve is rotated relative to the second sleeve.

7. The system of one of claims 1 to 6, wherein the first sleeve (1', 1 ") comprises a first end (1a) and an opposite second end (1b) and wherein the first sleeve (1', 1") comprises a longitudinal recess(17a, 17b) that extends in an axial direction and that is open to the second end, the width of the recess preferably being adapted to the width of the channel (204) for the rod of the receiving part (200', 200").

8. The system of one of claims 1 to 7, wherein the second sleeve (2', 2", 2"') comprises a first end (2a) and an opposite second end (2b) and wherein the second sleeve comprises a longitudinal recess (24a, 24b) that extends in an axial direction and that is open to the second end, the width of recess preferably being adapted ot the width of the channel (204) for the rod of the receiving part.

9. A spinal stabilization system for use with minimally invasive surgery comprising a system of claim 1 with at least two bone anchors and an extension device for each bone anchor according to one of claims 1 to 8.

## Patentansprüche

1. Ein System aus einer Verlängerungsvorrichtung und einem Knochenanker, wobei der Knochenanker umfasst:
einen Verankerungsabschnitt (100) zum Verankern in einem Knochen, und
ein Aufnahmeteil (200', 200"), das mit dem Verankerungsabschnitt verbunden ist, wobei das Aufnahmeteil eine Mittenachse (C) und einen Kanal (204) zum Aufnehmen eines Stabs umfasst, wobei Seitenwände des Kanals zwei freie Schenkel (205a, 205b) ausbilden, und
wobei die Verlängerungsvorrichtung umfasst:
eine erste Hülse (1', 1") mit einer ersten Hülsenachse (c), die mit der Mittenachse (C) koaxial ist, wobei die erste Hülse (1', 1") eingerichtet ist, entfernbar mit dem Aufnahmeteil (200', 200") und dieses kontaktierend verbunden zu werden, und
eine zweite Hülse (2', 2", 2"') mit einer zweiten Hülsenachse, wobei die zweite Hülse innerhalb der ersten Hülse (1', 1") positioniert und eingerichtet ist, mit dem Aufnahmeteil gekoppelt zu werden, um eine Drehbewegung der zweiten Hülse (2', 2", 2"') relativ zu dem Aufnahmeteil (200', 200") zu erschweren oder zu verhindern, wobei die zweite Hülse (2', 2", 2"') mit der ersten Hülse (1, 1') durch ein Kopplungselement (3) verbunden ist, das eingerichtet ist, in der ersten Hülse (1, 1') zusammen mit der zweiten Hülse (2, 2', 2", 2"') in einer axialen Richtung vorgetrieben zu werden,
wobei die erste Hülse (1', 1") einen sich umfänglich erstreckenden Vorsprung (120a, 120b) an einer inneren Oberfläche derselben umfasst, die eingerichtet ist, mit einer komplementären Struktur einer sich umfänglich erstreckenden Ausnehmung (206a, 206b) an einer äußeren Oberfläche des Aufnahmeteils (200', 200") in Eingriff zu treten, um einer translatorischen Bewegung der ersten Hülse (1', 1 ") relativ zum Aufnahmeteil entlang der Mittenachse vorzubeugen,
wobei die erste Hülse (1', 1 ") ferner wenigstens eine(n) sich in Längsrichtung erstreckende Rippe (112a, 112b) oder Vorsprung (129a, 129b) aufweist, die/der in eine entsprechende, sich in Längsrichtung erstreckende Ausnehmung (212a, 212b, 210a, 210b) eingreift, die an einem der Schenkel des Aufnahmeteils (200', 200") ausgebildet ist, um auch einer Drehbewegung des Aufnahmeteils relativ zu der ersten Hülse (1', 1") vorzubeugen,
**dadurch gekennzeichnet, dass** die sich in Längsrichtung erstreckenden Ausnehmungen (212a, 212b, 210a, 210b) die sich umfänglich erstreckenden Ausnehmungen (206a, 206b) schneiden, um eine formschlüssige Verbindung zwischen der ersten Hülse (1', 1") und dem Aufnahmeteil (200', 200") herzustellen.

2. Das System gemäß Anspruch 1, wobei das Kopplungselement (3) mit der ersten Hülse (1', 1") durch eine Vortriebsstruktur (33, 16) gekoppelt ist, die es erlaubt, das Kopplungselement mit der zweiten Hülse (2', 2", 2"') zu einer vorgegebenen Position vorzutreiben.

3. Das System gemäß Anspruch 1 oder 2, wobei das Kopplungselement (3) eine Lagerhülse ist und die Vortriebsstruktur ein Gewinde (33, 16) umfasst.

4. Das System gemäß einem der Ansprüche 1 bis 3, wobei das Kopplungselement (3) mit der zweiten Hülse (2', 2", 2"') über einen Anschlag (21a) der zweiten Hülse (2', 2", 2"') gekoppelt ist, gegen welchen das Kopplungselement (3) anschlägt, wobei eine Drehung des Kopplungselements (3) in Bezug auf die zweite Hülse (2', 2", 2"') ermöglicht ist.

5. Das System gemäß einem der Ansprüche 1 bis 4, wobei die zweite Hülse (2", 2"') einen Vorsprung (27a, 27b; 270a, 270b) und oder eine Ausnehmung (290a, 290b) an einer Oberfläche derselben umfasst, die eingerichtet ist, mit einer komplementären Struktur an einer Oberfläche des Aufnahmeteils in Eingriff zu treten, um eine formschlüssige Verbindung zwischen der zweiten Hülse und dem Aufnahmeteil herzustellen.

6. Das System gemäß einem der Ansprüche 1 bis 5, wobei die zweite Hülse (2') einen im Wesentlichen flachen Oberflächenabschnitt (26a', 26b') umfasst, der eingerichtet ist, mit einem im Wesentlichen flachen Oberflächenabschnitt (200a) an dem Aufnahmeteil (200') in Eingriff zu treten, so dass eine Reibung zwischen den Oberflächenabschnitten erzeugt wird, wenn die erste Hülse relativ zu der zweiten Hülse gedreht wird.

7. Das System gemäß einem der Ansprüche 1 bis 6, wobei die erste Hülse (1', 1") ein erstes Ende (1a) und ein gegenüberliegendes zweites Ende (1b) umfasst, und wobei die erste Hülse (1', 1 ") eine Längsausnehmung (17a, 17b) umfasst, die sich in einer axialen Richtung erstreckt und die zu dem zweiten Ende hin offen ist, wobei die Breite der Ausnehmung vorzugsweise an die Breite des Kanals (204) des Aufnahmeteils (200', 200") für den Stab angepasst ist.

8. Das System gemäß einem der Ansprüche 1 bis 7, wobei die zweite Hülse (2', 2", 2"') ein erstes Ende (2a) und ein gegenüberliegendes zweites Ende (2b) umfasst, und wobei die zweite Hülse eine Längsausnehmung (24a, 24b) umfasst, die sich in einer axialen Richtung erstreckt und die zu dem zweiten Ende hin offen ist, wobei die Breite der Ausnehmung vorzugsweise an die Breite des Kanals (204) des Aufnahmeteils für den Stab angepasst ist.

9. Ein Wirbelsäulenstabilisierungssystem zur Verwendung in minimal-invasiver Chirurgie, umfassend ein System gemäß Anspruch 1 mit wenigstens zwei Knochenankern und einer Verlängerungsvorrichtung für jeden Knochenanker entsprechend einem der Ansprüche 1 bis 8.

## Revendications

1. Système de dispositif d'extension et d'ancrage osseux,
dans lequel l'ancrage osseux comprend:
une section d'ancrage (100) destinée à être ancrée dans un os; et
une partie de réception (200', 200") connectée à la section d'ancrage, la partie de réception comprenant un axe central (C) et un canal (204) destiné à recevoir une tige, dans lequel des parois latérales du canal forment deux branches libres (205a, 205b), et
dans lequel le dispositif d'extension comprend:
un premier manchon (1', 1") présentant un premier axe de manchon (c) qui est coaxial à l'axe central (C), dans lequel le premier manchon (1', 1") est configuré de manière à entrer en contact avec et à être couplé de façon détachable à la partie de réception (200', 200"), et
un second manchon (2', 2", 2"') présentant un second axe de manchon, dans lequel le second manchon est positionné à l'intérieur du premier manchon (1', 1") et est configuré de manière à être couplé à la partie de réception de manière à entraver ou à empêcher un déplacement rotatif du second manchon (2', 2", 2"') par rapport à la partie de réception (200', 200"), dans lequel le second manchon (2', 2", 2"') est connecté au premier manchon (1, 1') par l'intermédiaire d'un élément de couplage (3) qui est configuré de manière à s'avancer dans le premier manchon (1', 1") de concert avec le second manchon (2', 2", 2"') dans une direction axiale;
dans lequel le premier manchon (1', 1") comporte une saillie s'étendant circonférentiellement (120a, 120b) au niveau d'une surface intérieure de celui-ci qui est configurée de manière à engager une structure complémentaire d'une rainure s'étendant circonférentiellement (206a, 206b) au niveau d'une surface extérieure de la partie de réception (200', 200") de manière à empêcher un déplacement translatif du premier manchon (1', 1") par rapport à la partie de réception le long de l'axe central,
dans lequel le premier manchon (1', 1") comporte en outre au moins une nervure (112a, 112b) ou une saillie (129a, 129b) s'étendant longitudinalement qui engage une rainure s'étendant longitudinalement correspondante (212a, 212b, 210a, 210b) formée au niveau de l'une des branches de la partie de réception (200', 200") de manière à empêcher également un déplacement rotatif de la partie de réception par rapport au premier manchon (1', 1 "),
**caractérisé en ce que** les rainures longitudinales (212a, 212b, 210a, 210b) coupent les rainures s'étendant circonférentiellement (206a, 206b) de manière à réaliser une connexion ajustée entre le premier manchon (1', 1") et la partie de réception (200', 200").

2. Système selon la revendication 1, dans lequel l'élément de couplage (3) est couplé au premier manchon (1', 1") par l'intermédiaire d'une structure d'avancement (33, 16) qui permet de faire avancer l'élément de couplage avec le second manchon (2', 2", 2"') jusqu'à une position donnée.

3. Système selon la revendication 1 ou 2, dans lequel l'élément de couplage (3) est une douille et la structure d'avancement comporte des filets (33, 16).

4. Système selon l'une des revendications 1 à 3, dans lequel l'élément de couplage (3) est couplé au second manchon (2', 2", 2"') par l'intermédiaire d'une butée (21a) du second manchon (2', 2", 2"') contre laquelle l'élément de couplage (3) bute, dans lequel une rotation de l'élément de couplage (3) par rapport au second manchon (2', 2", 2"') est autorisée.

5. Système selon l'une des revendications 1 à 4, dans lequel le second manchon (2", 2"') comporte une saillie (27a, 27b; 270a, 270b) et/ou un évidement (290a, 290b) au niveau d'une surface de celui-ci et qui est configurée de manière à engager une structure complémentaire au niveau d'une surface de la partie de réception afin de réaliser une connexion ajustée entre le second manchon et la partie de réception.

6. Système selon l'une des revendications 1 à 5, dans lequel le second manchon (2') présente une partie de surface sensiblement plate (26a', 26b') qui est configurée de manière à engager une partie de surface sensiblement plate (200a) au niveau de la partie de réception (200') de telle sorte qu'un frottement entre les parties de surface soit généré lorsque le premier manchon est tourné par rapport au second manchon.

7. Système selon l'une des revendications 1 à 6, dans lequel le premier manchon (1', 1") présente une première extrémité (1a) et une seconde extrémité opposée (1b), et dans lequel le premier manchon (1', 1") comporte un évidement longitudinal (17a, 17b) qui s'étend dans une direction axiale et qui est ouvert vers la seconde extrémité, la largeur de l'évidement étant de préférence adaptée à la largeur du canal (204) pour la tige de la partie de réception (200', 200").

8. Système selon l'une des revendications 1 à 7, dans lequel le second manchon (2', 2", 2"') présente une première extrémité (2a) et une seconde extrémité opposée (2b), et dans lequel le second manchon comporte un évidement longitudinal (24a, 24b) qui s'étend dans une direction axiale et qui est ouvert vers la seconde extrémité, la largeur de l'évidement étant de préférence adaptée à la largeur du canal (204) pour la tige de la partie de réception.

9. Système de stabilisation de la colonne vertébrale destiné à être utilisé en chirurgie minimalement invasive, et comprenant un système selon la revendication 1 présentant au moins deux ancrages osseux et un dispositif d'extension pour chaque ancrage osseux selon l'une des revendications 1 à 8.
